# EUROPEAN PATENT APPLICATION

(11) **EP 2 989 969 A1**
(43) Date of publication of application: **02.03.2016**
(21) Application number: 15174278.0
(22) Date of filing: 29.06.2015
(51) Int. Cl.: A61B 5/00

(54) **PROBE FOR AN OPTOACOUSTIC IMAGING DEVICE**

(30) Priority: 29.08.2014 JP 2014175465
(71) Applicant: PreXion Corporation, Chiyoda-ku Tokyo 101-0041 (JP)
(72) Inventor: SATO, Naoto, Chiyoda-ku, Tokyo 101-0041 (JP); MORISONO, Koji, Chiyoda-ku, Tokyo 101-0041 (JP); AGANO, Toshitaka, Chiyoda-ku, Tokyo 101-0041 (JP)
(74) Representative: Grünecker Patent- und Rechtsanwälte PartG mbB

(57) **Abstract**

A probe for an optoacoustic imaging device has an irradiator and a detector. The irradiator includes a light-emitting semiconductor element light source that irradiates a tested object with light. The detector detects an optoacoustic wave generated in the tested object as a result. The irradiator is removably fitted to the detector.

## Description

### BACKGROUND OF THE INVENTION

### 1. Field of the Invention

The present invention relates to probes for optoacoustic imaging devices.

### 2. Description of Related Art

Optoacoustic imaging is known whereby an optoacoustic wave which is an elastic wave generated as a result of light transmitted from a light source into a tested object being absorbed inside the tested object is detected and turned into an image through signal processing.

For example, Japanese patent application published No. 2013-233238 (hereinafter "Patent Document 1") discloses a probe for an optoacoustic imaging device, and this probe comprises an optical fiber for transmitting laser light emitted from a laser light source and a light guide member for guiding the laser light transmitted across the optical fiber to a tested object.

Inconveniently, however, systems employing lasers are large in size, and solid-state lasers are expensive; thus systems employing LED light sources are sought. An LED light source may be applied to the probe disclosed in Patent Document 1, but then, to allow the use of different types of LED light sources (having different wavelengths, etc.), as many probes need to be built, which is disadvantageous in terms of cost, etc.

Incidentally, Japanese patent application published No. 2013-48892 (hereinafter "Patent Document 2") discloses a probe provided with an attachment having a light guide member for guiding laser light emitted from a laser light source to a tested object. However, Patent Document 2 gives no consideration to using an LED light source.

### SUMMARY OF THE INVENTION

An object of the present invention is to provide a probe for an optoacoustic imaging device which allows use of a plurality of kinds of light-emitting semiconductor element light sources without requiring an increased number of probes and which are thus advantageous in terms of cost, etc.

To achieve the above object, according to one aspect of the present invention, a probe for an optoacoustic imaging device includes: an irradiator including a light-emitting semiconductor element light source that irradiates a tested object with light; and a detector which detects an optoacoustic wave generated in the tested object as a result. Here, the irradiator is removably fitted to the detector.

The probe structured as described above may further include: a slide mechanism which enables the sliding of the irradiator relative to the detector; and a lock which locks at the slide position up to which the sliding enabled by the slide mechanism is limited.

The probe structured as described above may further include: a fastening mechanism which fastens the irradiator to the detector. The fastening mechanism includes two first hooks so biased as to come close together by an elastic member and a second hook. Here, inserting the second hook between the first hooks causes the first hooks to come apart and lock the second hook. The fastening mechanism may further include an elevation and a depression which engage with each other when the second hook is locked by the first hooks.

The probe structured as described above may further include: releasable clamps which in a closed state hold the detector from opposite sides, and a lock which locks the releasable clamps in the closed state.

The probe structured as described above may further include: an attachment which is removably fitted to the detector. Here, the irradiator is removably fitted to the attachment.

The probe structured as described above may further include: a slide mechanism which enables the sliding of the irradiator relative to the attachment; and a lock which locks at the slide position up to which the sliding enabled by the slide mechanism is limited.

The probe structured as described above may further include: a fastening mechanism which fastens the irradiator to the detector. The fastening mechanism includes two first hooks so biased as to come close together by an elastic member and a second hook. Here, inserting the second hook between the first hooks causes the first hooks to come apart and lock the second hook. The fastening mechanism may further include an elevation and a depression which engage with each other when the second hook is locked by the first hooks.

In the probe structured as described above, the irradiator may include: a first cover having an inner surface that, when the irradiator is fitted on a tip end part of the detector, makes close contact with the tip end portion; and a second cover having a lock that, when the irradiator is fitted on the tip end part of the detector, locks on the tip end portion. Here, the light-emitting semiconductor element light source is arranged around the circumference of the first cover while being enclosed by the first and second covers.

In any of the probes structured as described above, a cable for supplying electric power from the main body of the optoacoustic imaging device to the light-emitting semiconductor element light source may be connected to the irradiator.

In any of the probes structured as described above, the detector may have a first connector, and the irradiator may have a second connector which, when the irradiator is fitted to the detector, couples with the first connector. Here, electric power is supplied via the first and second connector to the light-emitting semiconductor element light source.

In any of the probes structured as described above, the irradiator may include: a memory which stores identification information based on which the light-emitting semiconductor element light source can be identified; a fitting detector which detects that the irradiator is fitted to the detector, and a transmitter which transmits the identification information based on the result of detection by the fitting detector.

### BRIEF DESCRIPTION OF THE DRAWINGS

Fig. 1 is an exploded perspective view of an optoacoustic probe (a probe for an optoacoustic imaging device) according to a first embodiment of the present invention;
Fig. 2 is a perspective view of the optoacoustic probe according to the first embodiment of the present invention;
Fig. 3 is a block configuration diagram of an optoacoustic imaging device according to the first embodiment of the present invention;
Fig. 4 is a side view of a tip end part of the ultrasonic probe according to the first embodiment of the present invention;
Fig. 5 is a perspective view of an upper light source cover according to the first embodiment of the present invention;
Fig. 6 comprises a rear view and a side view of the upper light source cover according to the first embodiment of the present invention;
Fig. 7 is an exploded perspective view of an optoacoustic probe according to a second embodiment of the present invention;
Fig. 8 is a perspective view of the optoacoustic probe according to the second embodiment of the present invention;
Fig. 9 is a rear perspective view of an upper light source cover according to the second embodiment of the present invention;
Fig. 10 is a front view of the upper light source cover according to the second embodiment of the present invention;
Fig. 11A is a sectional view across line B-B in Fig. 10;
Fig. 11B is a sectional view showing hooks locked from the state shown in Fig. 11 A;
Fig. 12 is an exploded perspective view of an optoacoustic probe according to a third embodiment of the present invention;
Fig. 13 is a perspective view of the optoacoustic probe according to the third embodiment of the present invention;
Fig. 14 is a perspective view of the optoacoustic probe according to the third embodiment of the present invention, with clamps swung open;
Fig. 15 is a top view of an upper light source cover according to the third embodiment of the present invention (with the clamps in a closed state, but unlocked);
Fig. 16 is a top view of the upper light source cover according to the third embodiment of the present invention (with the clamps in an open state);
Fig. 17 is a top view of the upper light source cover according to the third embodiment of the present invention (with the clamps in a closed state, and locked);
Fig. 18 is a perspective view of a part of an optoacoustic probe according to a fourth embodiment of the present invention;
Fig. 19 is a top view showing a lock mechanism according to the fourth embodiment of the present invention;
Fig. 20 is a top view showing the lock mechanism according to the fourth embodiment of the present invention;
Fig. 21 is a top view showing the lock mechanism according to the fourth embodiment of the present invention;
Fig. 22 is an exploded perspective view of an optoacoustic probe according to a fifth embodiment of the present invention;
Fig. 23 is an exploded perspective view of an optoacoustic probe according to a sixth embodiment of the present invention;
Fig. 24 is an exploded perspective view of an optoacoustic probe according to a seventh embodiment of the present invention;
Fig. 25 is a present invention showing how an irradiation unit is fitted in the ultrasonic probe according to the seventh embodiment of the present invention;
Fig. 26 is a perspective view showing the rear face of the irradiation unit according to the seventh embodiment of the present invention;
Fig. 27 is a side view showing the irradiation unit according to the seventh embodiment of the present invention fitted on an ultrasonic wave probe;
Fig. 28 is a perspective view of a part of an optoacoustic probe according to an eighth embodiment of the present invention;
Fig. 29 is an exploded perspective view of an optoacoustic probe according to a ninth embodiment of the present invention; and
Fig. 30 is a perspective view showing, in outline, a part of an optoacoustic probe according to a tenth embodiment of the present invention.

### DETAILED DESCRIPTION OF PREFERRED EMBODIMENTS

### First Embodiment

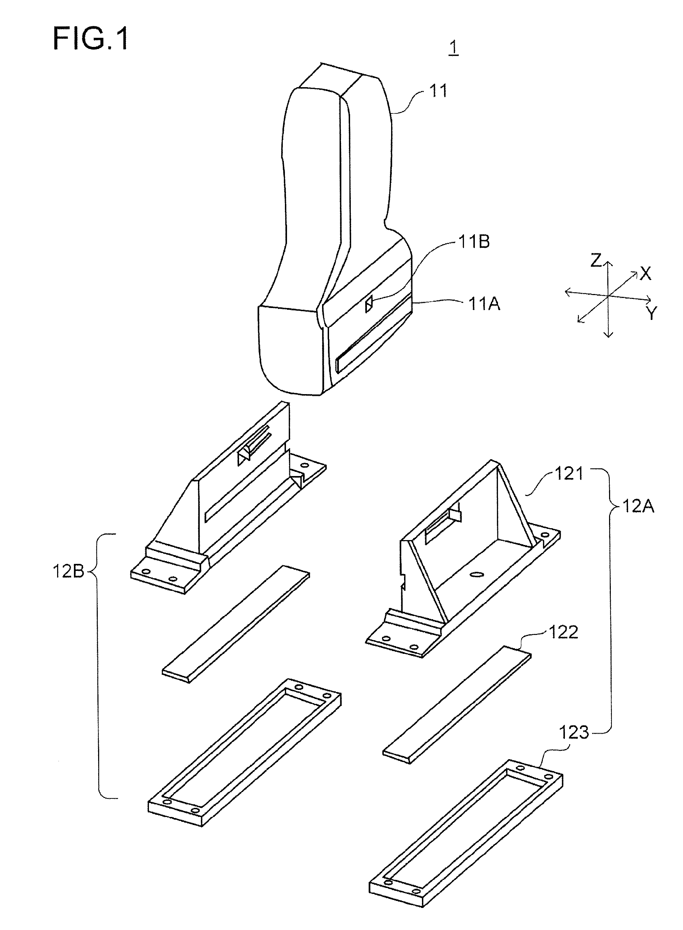

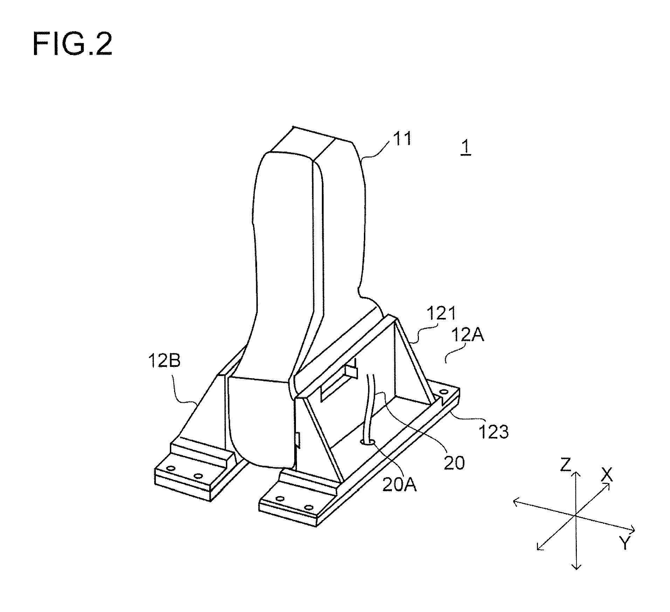

Hereinafter, embodiments of the present invention will be described with reference to the accompanying drawings. First, a first embodiment of the present invention will be described. An explosive perspective view of a probe for an optoacoustic imaging device (hereinafter "optoacoustic probe") according to the first embodiment is shown in Fig. 1, and a perspective view of the optoacoustic probe put together is shown in Fig. 2. The configuration of an optoacoustic imaging device provided with an optoacoustic probe embodying the present invention will be described later.

The optoacoustic probe 1 shown in Figs. 1 and 2 includes an ultrasonic wave probe 11 which emits an ultrasonic wave into a tested object (living body) and detects the ultrasonic wave reflected inside the tested object, and irradiation units 12A and 12B which irradiate the tested object with light. Here, the ultrasonic wave probe 11 is assumed to be of a so-called linear type, but this is not meant as any limitation; it may instead be of a convex type or of a sector type.

The irradiation units 12A and 12B are each provided with an upper light source cover 121, an LED light source 122, and a lower light source cover 123. The LED light source 122 is mounted on the lower light source cover 123, and for the sake of protection, the lower light source cover 123 is sealed by the upper light source cover 121. That is, the LED light source 122 is housed inside a cover composed of the upper light source cover 121 and the lower light source cover 123. Each structured as described above, the irradiation units 12A and 12B are arranged on the front and rear sides, respectively, of the ultrasonic wave probe 11 so as to hold the ultrasonic wave probe 11 from opposite sides, and are fastened to the ultrasonic wave probe 11. How the fastening is achieved will be described in detail later.

As shown in Fig. 2, the upper light source cover 121 is provided with a hole 20A through which to pass a power cable 20, and through the hole 20A, the power cable 20 is connected to the LED light source 122. Across the power cable 20, the LED light source 122 is supplied with electric power. The other end of the power cable 20 is connected to the main body (unillustrated) of an optoacoustic imaging device.

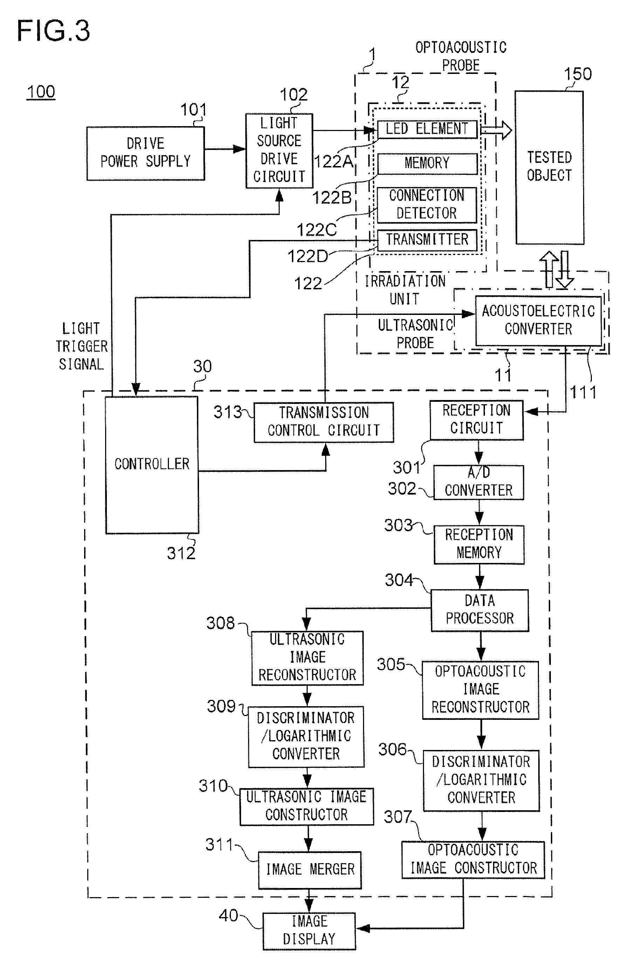

A block configuration of an optoacoustic imaging device embodying the present invention, provided with the optoacoustic probe 1, is shown in Fig. 3. The optoacoustic imaging device 100 shown in Fig. 3 includes, in addition to the optoacoustic probe 1, a drive power supply 101, a light source drive circuit 102, an image generator 30, and an image display 40. The drive power supply 101, the light source drive circuit 102, the image generator 30, and the image display 40 are provided, for example, in the main body (unillustrated) of the optoacoustic imaging device 100.

In Fig. 3, for convenience' sake, the irradiation units 12A and 12B included in the optoacoustic probe 1 are collectively identified as an irradiation unit 12. The irradiation unit 12 includes an LED light source 122, which has an LED element 122A mounted on a substrate. The LED element 122A may comprise a single element, or a plurality of elements connected in series and/or in parallel.

The ultrasonic wave probe 11 (Fig. 2) included in the optoacoustic probe 1 incorporates an acoustoelectric converter 111. The acoustoelectric converter 111 is composed of a plurality of unillustrated ultrasonic oscillating elements arrayed in the left-right direction as seen from in front (in Fig. 2, the X direction) near a tip end part of the ultrasonic wave probe 11 which is pressed against the surface of the tested object 150.

The ultrasonic oscillating elements are piezoelectric elements which, when a voltage is applied to them, oscillate and generate an ultrasonic wave and which, when vibration (ultrasonic wave) is applied to them, generates a voltage. Between the acoustoelectric converter 111 and the surface of the tested object 150, an adjustment layer (unillustrated) is provided to allow adjustment of a difference in acoustic impedance. The adjustment layer serves to propagate the ultrasonic wave generated by the ultrasonic oscillating elements efficiently into the tested object 150, and also serves to propagate the ultrasonic wave (including an optoacoustic wave) from inside the tested object 150 to the ultrasonic oscillating elements.

The light source drive circuit 102 is supplied with electric power from the drive power supply 101. The LED element 122A emits pulsating light by being driven with a drive signal fed from the light source drive circuit 102, and irradiates the tested object 150 with LED light. The drive signal is fed from the light source drive circuit 102 to the LED light source 122 across the power cable 20 (Fig. 2).

The pulsating light emitted from the LED element 122A passes through the lower light source cover 123 (Fig. 1) and enters the tested object 150 while being diffused, and is absorbed by a light absorber (living tissue) inside the tested object 150. When the light absorber absorbs light, adiabatic expansion occurs, whereby an optoacoustic wave (ultrasonic wave), which is an elastic wave, is generated. The generated optoacoustic wave propagates inside the tested object 150, and is converted into a voltage signal by the ultrasonic oscillating elements (acoustoelectric converter 111).

The ultrasonic oscillating elements (acoustoelectric converter 111) also generate an ultrasonic wave to transmit it into the tested object 150, and receives the ultrasonic wave reflected inside the tested object 150 to generate a voltage signal. Thus, the optoacoustic imaging device 100 embodying the present invention can perform not only optoacoustic imaging but also ultrasonic imaging.

The image generator 30 includes a reception circuit 301, an A/D converter 302, a reception memory 303, a data processor 304, an optoacoustic image reconstructor 305, a discriminator/logarithmic converter 306, an optoacoustic image constructor 307, an ultrasonic image reconstructor 308, a discriminator/logarithmic converter 309, an ultrasonic image constructor 310, a image merger 311, a controller 312, and a transmission control circuit 313.

The reception circuit 301 selects, out of the plurality of ultrasonic oscillating elements, a part of them, and amplifies the voltage signal (detection signal) with respect to the selected ultrasonic oscillating elements.

In optoacoustic imaging, for example, the plurality of ultrasonic oscillating elements are divided into two regions adjoining in the left-right direction as seen from in front (in Fig. 2, the X direction); of the two regions, one is selected for first-time irradiation, and the other is selected for second-time irradiation. In ultrasonic imaging, for example, an ultrasonic wave is generated while switching is performed from one part of the plurality of ultrasonic oscillating elements to another, i.e., from one group of adjoining ultrasonic oscillating elements to another (so-called linear electronic scanning), and the reception circuit 301 accordingly so switches as to select one group after another.

The A/D converter 302 converts the amplified detection signal from the reception circuit 301 into a digital signal. The reception memory 303 stores the digital signal from the A/D converter 302. The data processor 304 serves to branch the signal stored in the reception memory 303 between the optoacoustic image reconstructor 305 and the ultrasonic image reconstructor 308.

The optoacoustic image reconstructor 305 performs phase matching addition based on the detection signal of an optoacoustic wave, and reconstructs the data of the optoacoustic wave. The discriminator/logarithmic converter 306 performs logarithmic compression and envelope discrimination on the data of the reconstructed optoacoustic wave. The optoacoustic image constructor 307 then converts the data that has undergone the processing by the discriminator/logarithmic converter 306 into pixel-by-pixel luminance value data. Specifically, optoacoustic image data (grayscale data) is generated as data comprising the luminance value at every pixel on the XZ plane in Fig. 2.

On the other hand, the ultrasonic image reconstructor 308 performs phase matching addition based on the detection signal of an ultrasonic wave, and reconstructs the data of the ultrasonic wave. The discriminator/logarithmic converter 309 performs logarithmic compression and envelope discrimination based on the data of the reconstructed ultrasonic wave. The ultrasonic image constructor 310 then converts the data that has undergone the processing by the discriminator/logarithmic converter 309 into pixel-by-pixel luminance value data. Specifically, ultrasonic image data (grayscale data) is generated as data comprising the luminance value at every pixel on the XZ plane in Fig. 2.

The image merger 311 merges the optoacoustic image data and the ultrasonic image data together to generate composite image data. The image merging here may be achieved by superimposing the optoacoustic image on the ultrasonic image, or by putting together the optoacoustic image and the ultrasonic imaging side by side (or one on top of the other). The image display 40 displays an image based on the composite image data generated by the image merger 311.

The image merger 311 may output the optoacoustic image data or the ultrasonic image data as it is to the image display 40.

The controller 312 feeds a light trigger signal to the light source drive circuit 102 to make it transmit a drive signal.

In response to an instruction from the controller 312, the transmission control circuit 313 transmits the drive signal to the acoustoelectric converter 111 to make it generate an ultrasonic wave. The controller 312 also controls the reception circuit 301, etc.

Here, the emission wavelength of the LED element 122A can be set at a wavelength in a near-infrared region, examples including 750 nm, 850 nm, 930 nm, and 1210 nm. For example, oxidized hemoglobin in blood exhibits a high absorptance for light of a wavelength of 750 nm, and reduced hemoglobin in blood exhibits a high absorptance for light of a wavelength of 850 nm. The wavelength of the LED element 122A may be the same between the irradiation units 12A and 12B, or may be different between them. For example, the irradiation unit 12A can be set at 750 nm, and the irradiation unit 12B at 850 nm.

The LED element 122A may comprise a combination of elements of a plurality of wavelengths. In that case, the light source drive circuit 102 transmits separate drive signals to LED elements of different wavelengths.

The LED light source 122 also has a memory 122B, a connection detector 122C, and a transmitter 122D mounted on the substrate, and these will be described later.

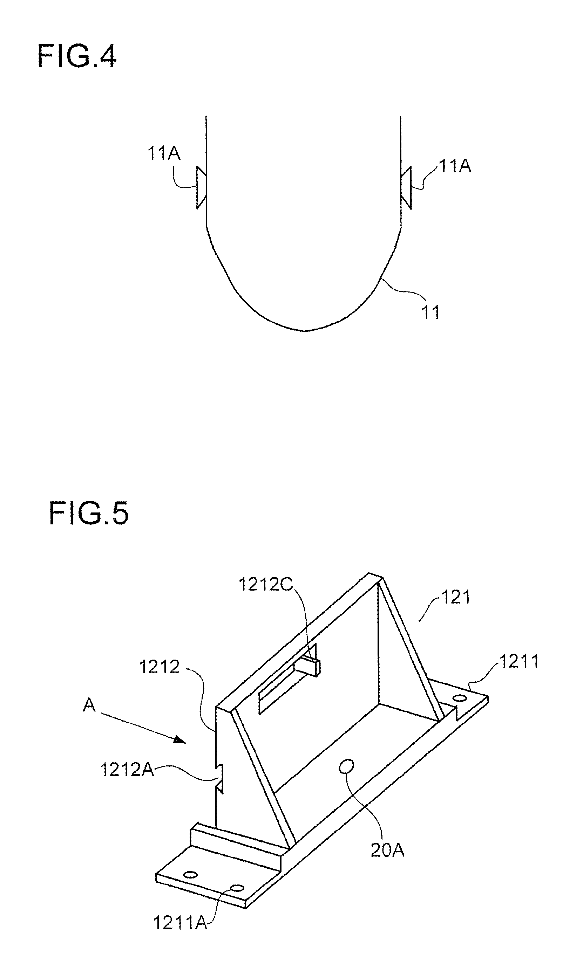

Next, how the irradiation units 12A and 12B are fitted to the ultrasonic wave probe 11 will be described with reference to Figs. 4 to 6.

As shown in Fig. 1, the front and rear faces of the housing of the ultrasonic wave probe 11 are each provided with an elevation 11A extending in the left-right direction (X direction) and a depression 11B in a rectangular shape (the rear face is not shown in Fig. 1). The elevation 11A extends substantially from one end to the opposite end of the ultrasonic wave probe 11. Moreover, as shown in Fig. 4, which is a side view of the ultrasonic wave probe 11, the elevation 11A is, as seen in side view, so shaped as to be increasingly wide from base to tip.

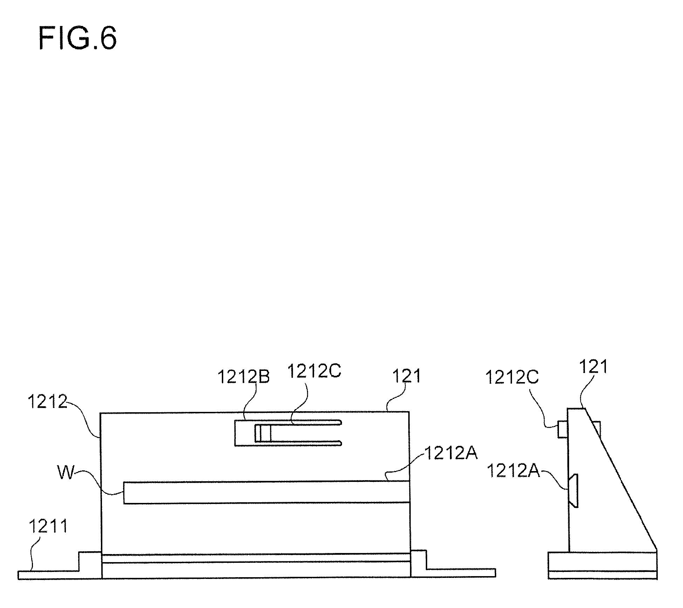

The structure of the upper light source cover 121 will now be described in detail with reference to Figs. 5 and 6. Fig. 5 is a perspective view of the upper light source cover 121, and Fig. 6 comprises a plan view (at left) as seen from direction A (from behind) in Fig. 5and a side view (at right) of the upper light source cover 121.

The upper light source cover 121 has a base 1211 and a wall 1212 protruding from the base 1211. In opposite end parts of the base 1211 in its longitudinal direction, holes 1211 A are formed respectively for fastening to the lower light source cover 123 (Fig. 1) with screws. The wall 1212 is provided with a depression 1212A extending in the longitudinal direction and a leaf spring-shaped hook 1212C formed inside a hole 1212B.

As shown in Fig. 6, the depression 1212A is formed to extend from one end of the upper light source cover 121 in its longitudinal direction to a position at a predetermined distance from the other end, and is, as seen in a side view, so shaped as to be increasingly wide inward of the upper light source cover 121.

The fitting of the irradiation unit 12A (or 12B) having the upper light source cover 121 structured as described above to the ultrasonic wave probe 11 can be achieved as follows. The elevation 11 A is engaged with the depression 1212A starting at a side end of the ultrasonic wave probe 11, and the upper light source cover 121 (irradiation unit) is slid along. Meanwhile, the leaf spring-shaped hook 1212C, in a state raised against a biasing force, slides across the surface of the ultrasonic wave probe 11.

The sliding proceeds until an end of the elevation 11A hits a wall W (Fig. 6) at an end of the depression 1212A and no further sliding is possible. Now, under the biasing force, the hook 1212C engages with the depression 11B. This prevents the upper light source cover 121 from coming loose. The removal of the irradiation unit can be achieved easily by raising the hook 1212C and sliding the upper light source cover 121 in the opposite direction.

As described above, according to this embodiment, the irradiation units 12A and 12B can be fitted to and removed from the ultrasonic wave probe 11 easily. Thus, a plurality of irradiation units having different types of LED light sources 122 (of different wavelengths, etc.) can be fitted to and removed from, and can thus interchangeably used with, a single ultrasonic wave probe 11. This is advantageous in terms of cost, etc.

As mentioned previously, in this embodiment, the memory 122B is mounted on the substrate of the LED light source 122. This memory 122B stores information on the characteristics of the LED element 122A; it stores, for example, information such as wavelength or combination of wavelengths, temperature coefficient, serial number, sealing resin thickness, presence or absence of a reflective plate, light intensity, forward voltage, rated current value, etc.

The connection detector (fitting detector) 122C can be configured, for example, to include a switch that is turned on when the upper light source cover 121 is slid until the hook 1212C engages with the depression 11B in the ultrasonic wave probe 11 and thus the irradiation unit is fastened. By detecting that the switch is on, the connection detector 122C can detect that an irradiation unit is connected to the optoacoustic probe 1.

In response to the detection of connection by the connection detector 122C, the transmitter 122D transits the information stored in the memory 122B to the controller 312. The transmission here may be achieved on a wired or wireless basis. Transmission on a wired basis can be achieved, for example, across the power cable 20.

Based on the information on the LED element 122A transmitted from the transmitter 122D, the controller 312 can perform various kinds of control. As one example, based on information on the intensity of the light transmitted from each of the irradiation units 12A and 12B, the controller 312 can control the amplification factor at the reception circuit 301 so as to compensate for a difference in light intensity.

### Second Embodiment

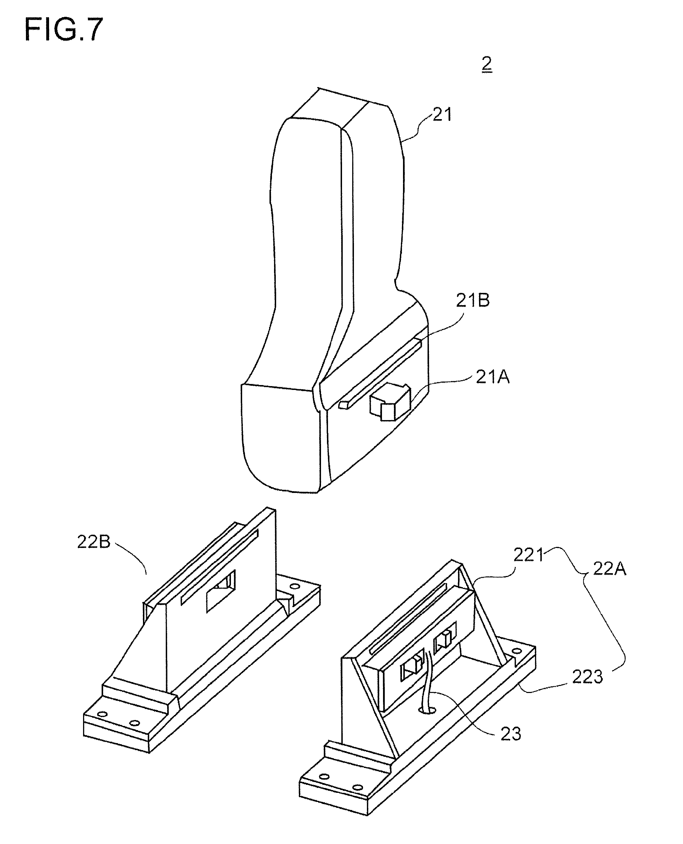

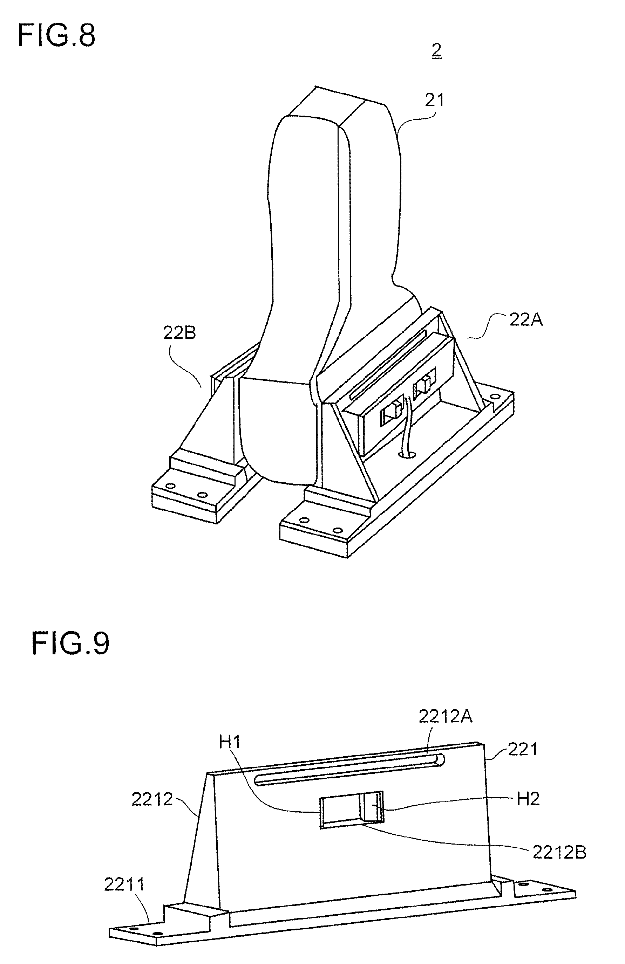

The first embodiment described above allows for various modifications, of which some examples will be described below, starting with a second embodiment of the present invention. A roughly exploded perspective view of an optoacoustic probe according to the second embodiment is shown in Fig. 7, and a perspective view of the optoacoustic probe put together is shown in Fig. 8.

The optoacoustic probe 2 shown in Figs. 7 and 8 includes an ultrasonic wave probe 21 and irradiation units 22A and 22B. The front and rear faces of the housing of the ultrasonic wave probe 21 are each provided with a hook 21A and a boss 21B for positioning. The irradiation unit 22A (and 22B) is composed of an upper light source cover 221, a lower light source cover 223, and an LED light source (unillustrated) housed inside a case composed of the former two.

Now, the structure of the upper light source cover 221 will be described in detail. Fig. 9 is a rear perspective view of the upper light source cover 221. The wall surface of a wall 2212 protruding from a base 2211 is provided with a positioning hole 2212A, which is an elongate hole extending in the longitudinal direction, and a fitting hole 2212B, into which the hook 21 A on the ultrasonic wave probe 21 is inserted. Further inward of the fitting hole 2212B, there are arranged hooks H1 and H2.

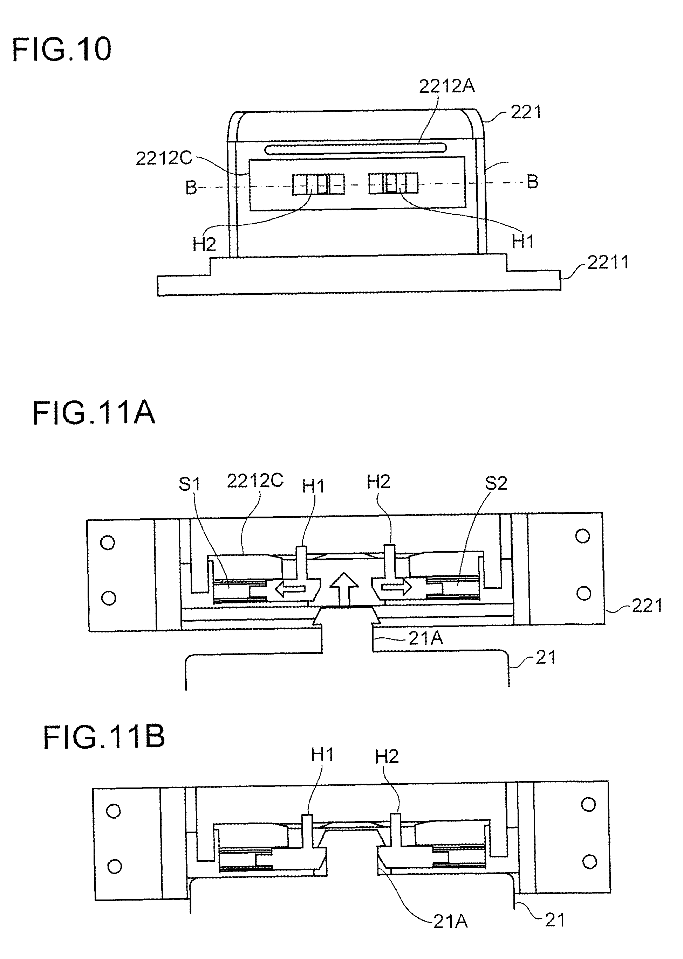

Fig. 10 is a front view of the upper light source cover 221 (as seen from behind what is shown in Fig. 9). A cover 2212C restricts the position of the hooks H1 and H2, and prevents them from coming loose.

Fig. 11A is a sectional view across line B-B in Fig. 10, and shows how the hook 21A on the ultrasonic wave probe 21 is inserted. The hooks H1 and H2 are biased toward the center by springs S1 and S2 respectively. As the hook 21A is inserted into the fitting hole 2212B, the hook 21A displaces the hooks H1 and H2 away from each other against the biasing forces of the springs S1 and S2. In a predetermined insertion position, the hook 21 A engages with the hooks H1 and H2, and is thereby prevented from coming loose (Fig. 11B). Now, the boss 21B engages with the positioning hole 2212A, and thereby achieves the positioning of the ultrasonic wave probe 21.

The removal of the ultrasonic wave probe 21 is achieved by pushing the hooks H1 and H2 away from each other with fingers or the like (at the front face shown in Fig. 10).

This embodiment provides similar effects as the first embodiment.

### Third Embodiment

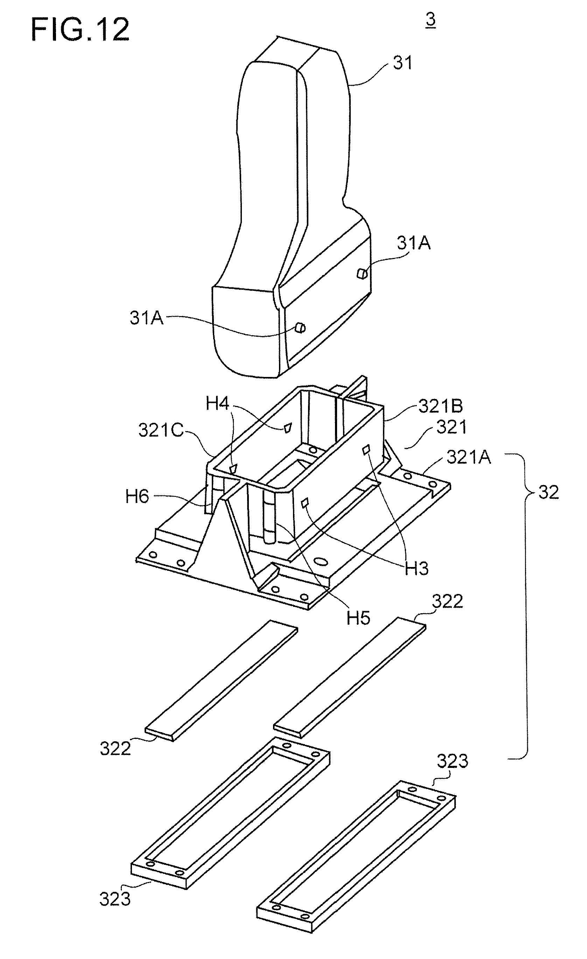

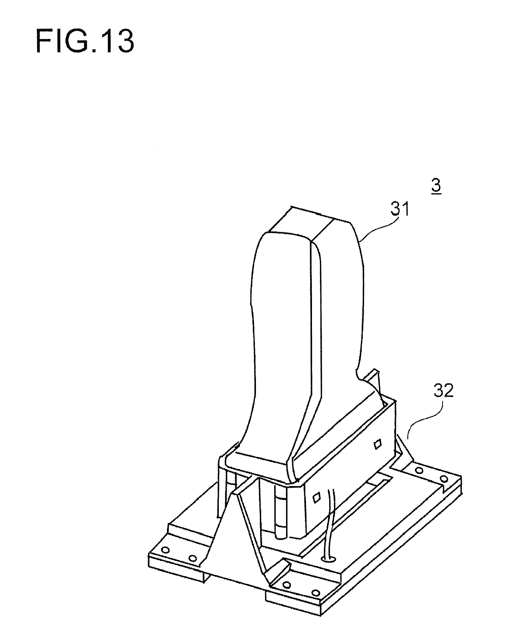

Next, a third embodiment of the present invention will be described. An explosive perspective view of an optoacoustic probe according to the third embodiment is shown in Fig. 12, and a perspective view of the optoacoustic probe put together is shown in Fig. 13.

The optoacoustic probe 3 shown in Figs. 12 and 13 includes an ultrasonic wave probe 31 and an irradiation unit 32. The irradiation unit 32 is composed of an upper light source cover 321, two LED light sources 322, and two lower light source covers 323. The LED light sources 322 are mounted on the lower light source covers 323 respectively, which are then sealed by the upper light source cover 321 to accommodate the LED light sources 322 inside.

The front and rear faces of the ultrasonic wave probe 31 are each provided with bosses 31A for positioning. The upper light source cover 321 includes a base 321A and clamps 321B and 321C, the latter being connected to the former so as to be pivotable about hinges H5 and H6.

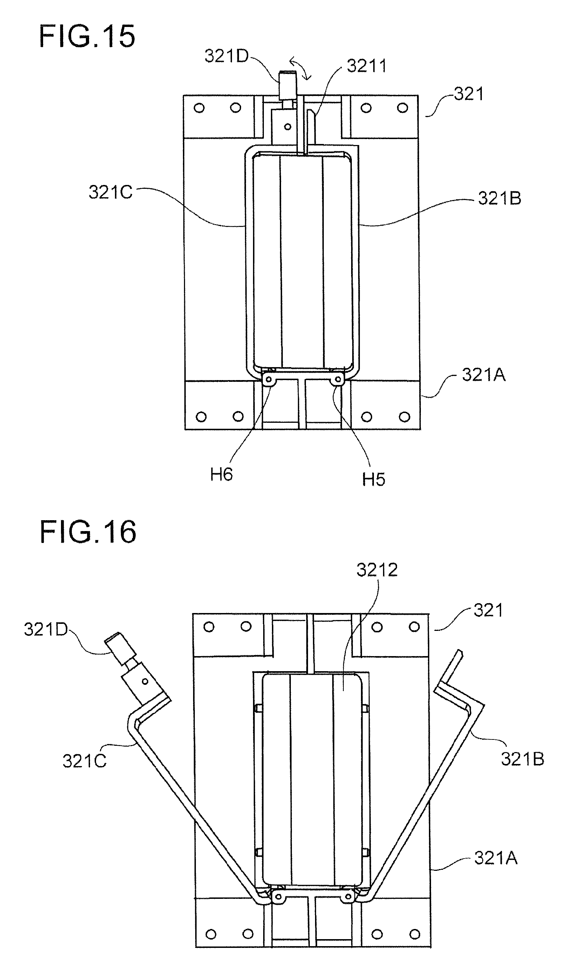

A top view of the upper light source cover 321 is shown in Fig. 15. As shown there, in an end part of the clamp 321C opposite from the hinge H6, a lock 321D is provided which is pivotable across 90 degrees as seen in a top view (indicated by arrows in Fig. 15) and which can also be turned as a screw. On the other hand, in an end part of the clamp 321 B opposite from the hinge H5, a tag 3211 is provided which has a cut formed in it.

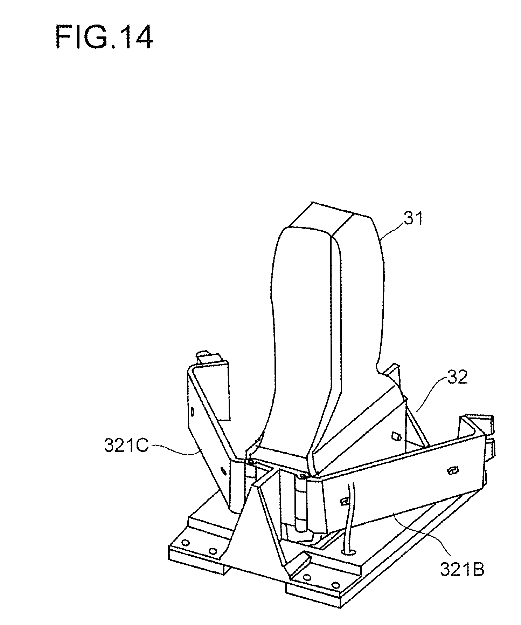

Here, the fitting of the ultrasonic wave probe 31 to the irradiation unit 32 is achieved as follows. First, as shown in Fig. 16, with the clamps 321B and 321C swung open, a tip end part of the ultrasonic wave probe 31 is pressed against a depression 3212 formed at the center of the base 321A. A perspective view of this state is shown in Fig. 14.

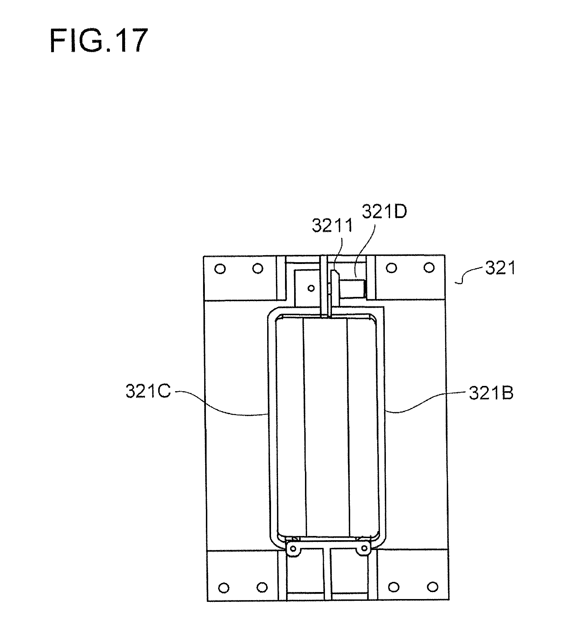

Then, as shown in Fig. 17, the clamps 321B and 321C are swung closed. This causes the bosses 31 A on the front face to engage with holes H3 (Fig. 12) formed in the clamp 321B and the bosses 31 A on the rear face to engage with the holes H4 formed in the clamp 321C. The lock 321D is then so swung that its shaft penetrates the cut in the tag 3211 to prevent the clamps 321B and 321C from swinging open. Further, the tip end part of the lock 321D is held and turned as a screw, so that the ultrasonic wave probe 11 is fastened tightly.

The removal of the ultrasonic wave probe 11 is achieved as follows. The tip end part of the lock 321D is held and turned as a screw to loosen screw-fastening, and is then swung 90 degrees as seen in a top view. Now the clamps 321B and 321C can be swung open so that the ultrasonic wave probe 11 can be removed.

This embodiment provides similar effects as the first embodiment.

### Fourth Embodiment

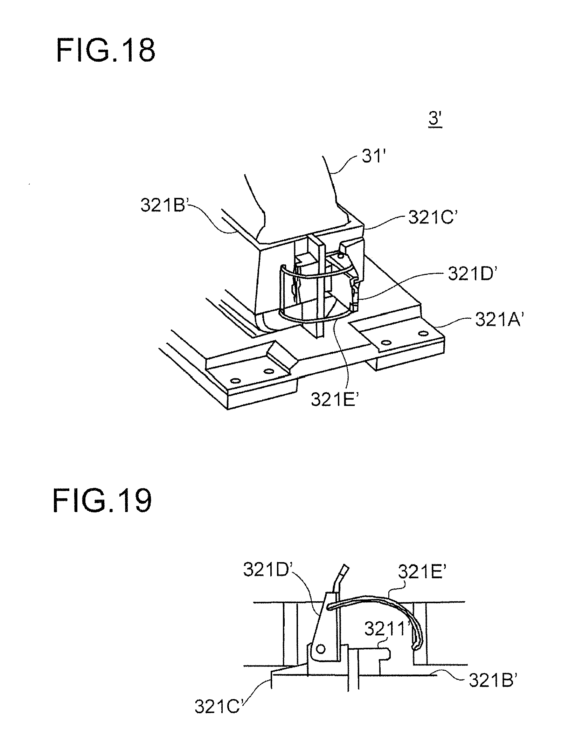

Next, a fourth embodiment of the present invention will be described. A perspective view of a part of an optoacoustic probe according to the fourth embodiment is shown in Fig. 18.

This embodiment is a modified example of the third embodiment described previously, and differs from it in the lock mechanism of the clamps. In the optoacoustic probe 3' shown in Fig. 18, clamps 321B' and 321C' are provided which are connected to the base 321A', on which the ultrasonic wave probe 31' is mounted, so as to be pivotable about hinges (unillustrated).

In one end part of the clamp 321C', a lever 321D' is pivotably connected, and to this lever 321D', a hook 321E' is pivotably connected.

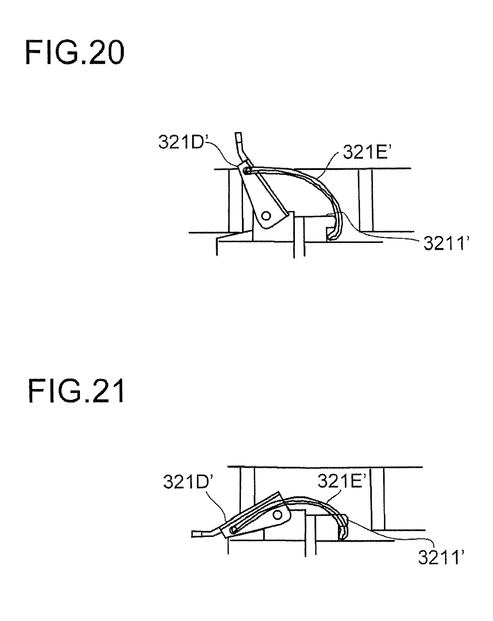

The ultrasonic wave probe 31' is mounted on the base 321A', and the clamps 321B' and 321C' are swung closed. This state is assumed to be as shown in a top view in Fig. 19. Then, as shown in Fig. 20, the hook 321E' is hung on a hook 3211' provided in an end part of the clamp 321B', and the lever 321D' is brought down. Now in a state as shown in Fig. 21, the clamps 321B' and 321C' are swung closed and fastened together, and accordingly the ultrasonic wave probe 31' is fastened.

This embodiment provides similar effects as the first embodiment.

### Fifth Embodiment

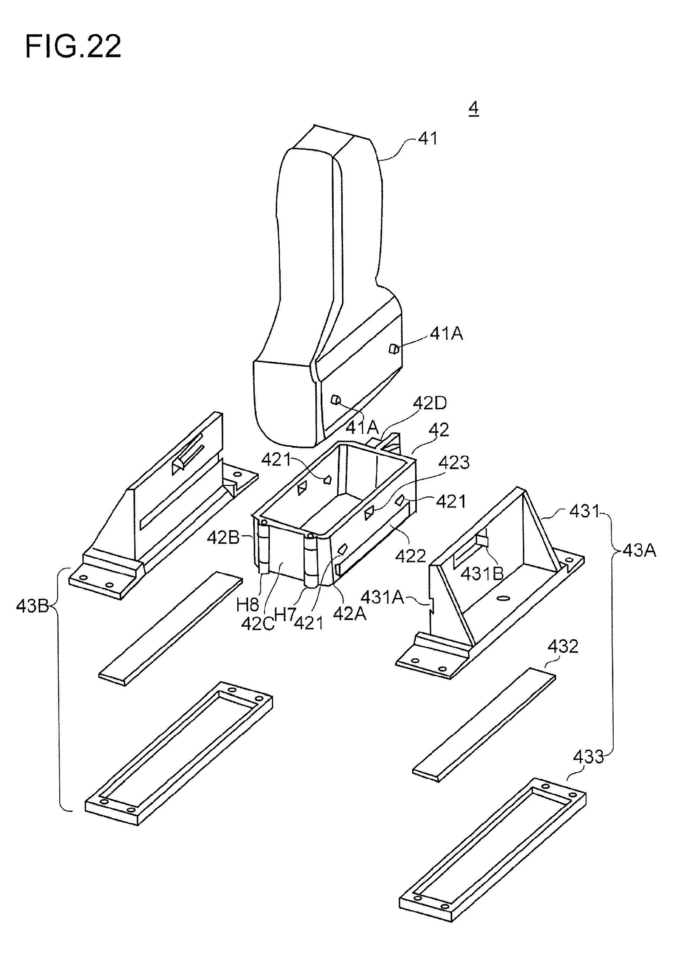

Next, a fifth embodiment of the present invention will be described. An exploded perspective view of an optoacoustic probe according to the fifth embodiment is shown in Fig. 22. The optoacoustic probe 4 shown in Fig. 22 includes an ultrasonic wave probe 41, an attachment 42, and irradiation units 43A and 43B.

The front and rear faces of the ultrasonic wave probe 41 are each provided with bosses 41 A. The attachment 42 has a structure similar to a part of the upper light source cover 321 (Fig. 12) in the third embodiment described previously; specifically, a clamp 42A is connected to a base 42C so as to be pivotable about a hinge H7, and a clamp 42B is connected to the base 42C so as to be pivotable about a hinge H8. The clamps 42A and 42B are provided with holes 421 with which the bosses 41A engage. The attachment 42 also has a lock 42D like the one in the third embodiment.

The fitting of the attachment 42 to the ultrasonic wave probe 41 is achieved as follows. The clamps 42A and 42B are swung open, and are then swung closed so as to hold the ultrasonic wave probe 41 from opposite sides so that the bosses 41 A engage the holes 421. Then, the clamps 42A and 42B are locked together with the lock 42D, and thus the ultrasonic wave probe 41 is fastened.

The lock on the attachment may instead be like the one described in connection with the fourth embodiment.

Moreover, the irradiation units 43A and 43B composed of an upper light source cover 431, an LED light source 432, and a lower light source cover 433, all structured as in the first embodiment, can be fitted to and removed from the attachment 42.

The fastening of the irradiation unit 43A (or 43B) to the attachment 42 is achieved in a similar manner as in the first embodiment. Specifically, with an elevation 422 formed on the clamp 42A (or 42B) engaged with the depression 431A formed in the upper light source cover 431, and the upper light source cover 431 is slid along. Then, a hook 431B on the upper light source cover 431 is engaged with a hole 423 formed in the clamp 42A (or 42B).

This embodiment provides similar effects as the first embodiment.

### Sixth Embodiment

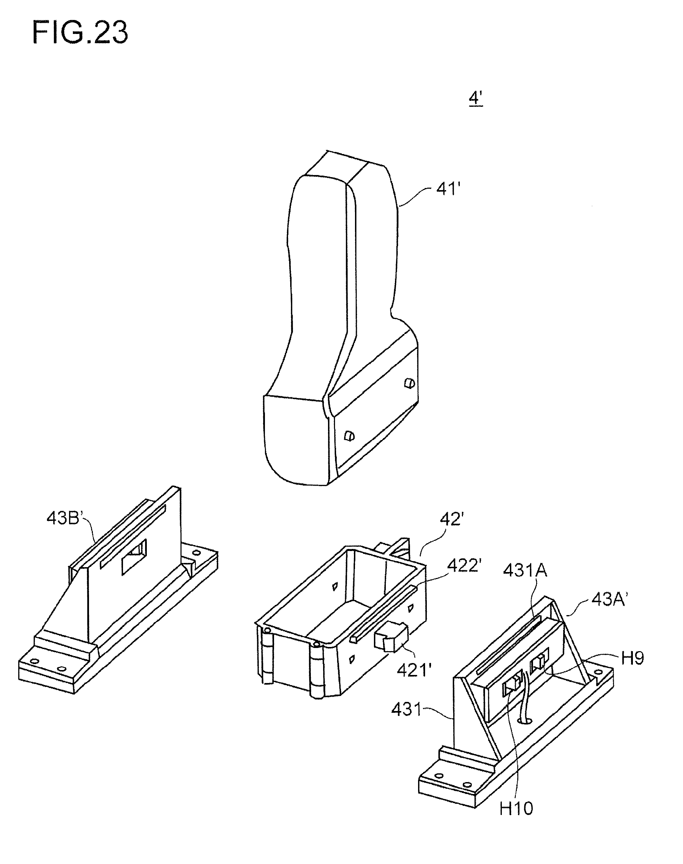

Next, a sixth embodiment of the present invention will be described. A roughly exploded perspective view of an optoacoustic probe according to the sixth embodiment is shown in Fig. 23. The optoacoustic probe 4' shown in Fig. 23 includes an ultrasonic wave probe 41', an attachment 42', and irradiation units 43A' and 43B'.

The structure for fastening the attachment 42' to the ultrasonic wave probe 41' is similar to that in the fifth embodiment (hence, the fastening is achieved in a similar manner). The irradiation units 43A' and 43B' structured in a similar manner as in the second embodiment can be fitted to and removed from the attachment 42'.

The fastening of the irradiation unit 43A' (or 43B') to the attachment 42' is achieved in a similar manner as in the second embodiment. Specifically, a hook 421' on the attachment 42' is inserted in a fitting hole (unillustrated) formed in the rear face of the upper light source cover 431. Then, hooks H9 and H10 are displaced away from each other against the biasing forces of springs to allow the hook 421' to be locked by the hooks H9 and H10. Now, a boss 422' on the attachment 42' engages with a positioning hole 431A.

This embodiment provides similar effect as the first embodiment.

### Seventh Embodiment

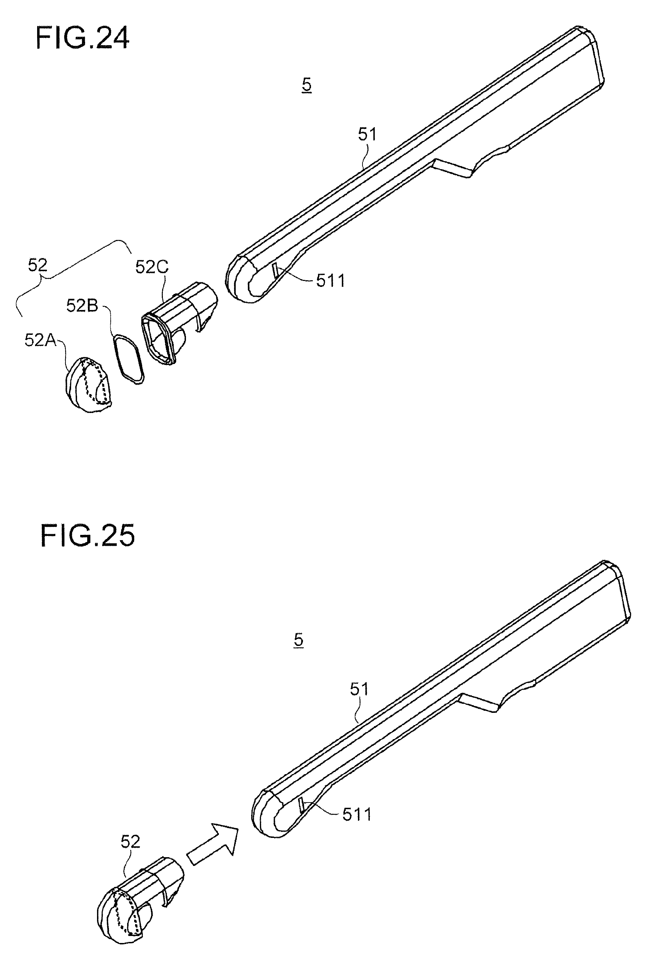

Next, a seventh embodiment of the present invention will be described. An exploded perspective view of an optoacoustic probe according to the seventh embodiment is shown in Fig. 24. The optoacoustic probe 5 shown in Fig. 24 includes an ultrasonic wave probe 51, which is a transrectal probe (or transvaginal probe), and an irradiation unit 52. The irradiation unit 52 is composed of an upper light source cover 52A, an LED light source 52B, and a lower light source cover 52C. As shown in Fig. 25, the irradiation unit 52 is fitted on a tip end part of the ultrasonic wave probe 51.

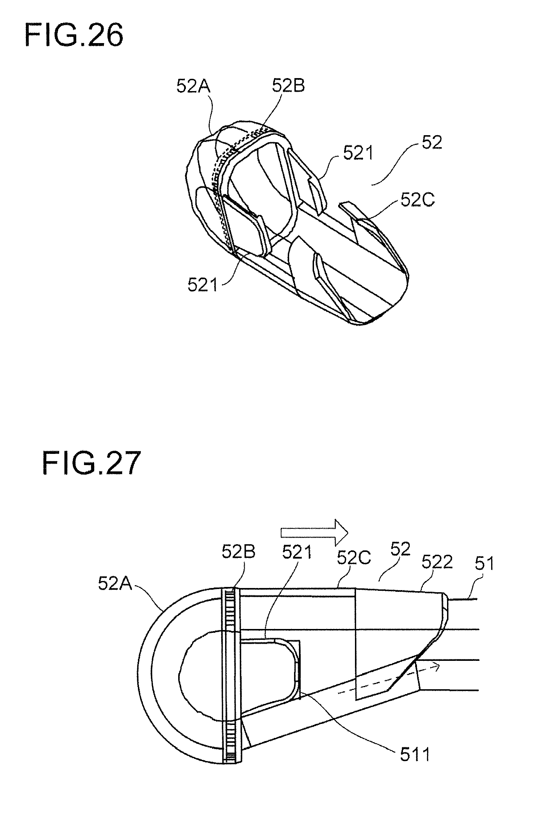

A perspective view of the irradiation unit 52 as seen from behind is shown in Fig. 26. Inside the upper light source cover 52A is a cavity into which a tip end part of the ultrasonic wave probe 51 is inserted. Around the circumference of the opening in the upper light source cover 52A through which the tip end part of the ultrasonic wave probe 51 is inserted, the LED light source 52B is arranged, and the upper light source cover 52A is sealed by the lower light source cover 52C. This protects the LED light source 52B. On the lower light source cover 52C, hooks 521 are provided opposite each other.

A side view of the irradiation unit 52 fitted on the tip end part of the ultrasonic wave probe 51 is shown in Fig. 27. In this state, the hooks 521 engage with depressions 511 provided in opposite sides of the tip end part of the ultrasonic wave probe 51, and serve to prevent the irradiation unit 52 from coming loose. The tip end part of the ultrasonic wave probe 51 is in close contact with the inner surface of the upper light source cover 52A.

The lower light source cover 52C has a sleeve 522 (Fig. 27) at its tip end; the tip end part of the ultrasonic wave probe 51 has an increasingly large diameter from base to tip, and the sleeve 522 is given a diameter smaller than the diameter of a base part of the tip end part of the ultrasonic wave probe 51. Thus, the base part is clasped by the sleeve 522, which thus tends to move in the direction indicated by a broken-line arrow in Fig. 27 to reduce the load. Consequently, the irradiation unit 52 is constantly acted on by a force acting in the fitting direction of the irradiation unit 52 (indicated by a solid-line arrow in Fig. 27). This too prevents the irradiation unit 52 from coming loose.

Also this embodiment allows easy fitting and removal of the irradiation unit 52 to and from the ultrasonic wave probe 51, and thus provides similar effects as the first embodiment.

### Eighth Embodiment

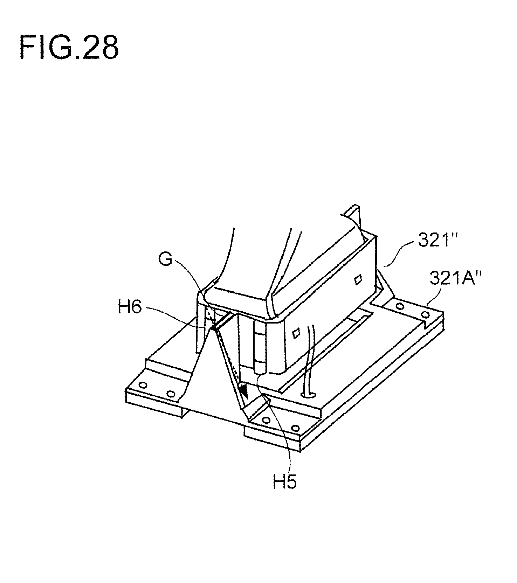

As a modified example of the third embodiment, as shown in Fig. 28, a puncture guide G may be additionally provided in the upper light source cover 321", so as to be contiguous with a part of the base 321A" between the hinges H5 and H6. This makes it possible to insert a puncture needle into the tested object via the puncture guide G. The puncture guide G may be so configured as to permit the needle insertion angle to be adjusted stepwise or continuously.

The attachments described in connection with the fifth and sixth embodiments may likewise be provided with a puncture guide.

### Ninth Embodiment

In the second embodiment (Fig. 7), a power cable 23 is connected to the irradiation unit 22A (or 22B) so that electric power is supplied from the main body of the optoacoustic imaging device via the power cable 23 to the LED light source.

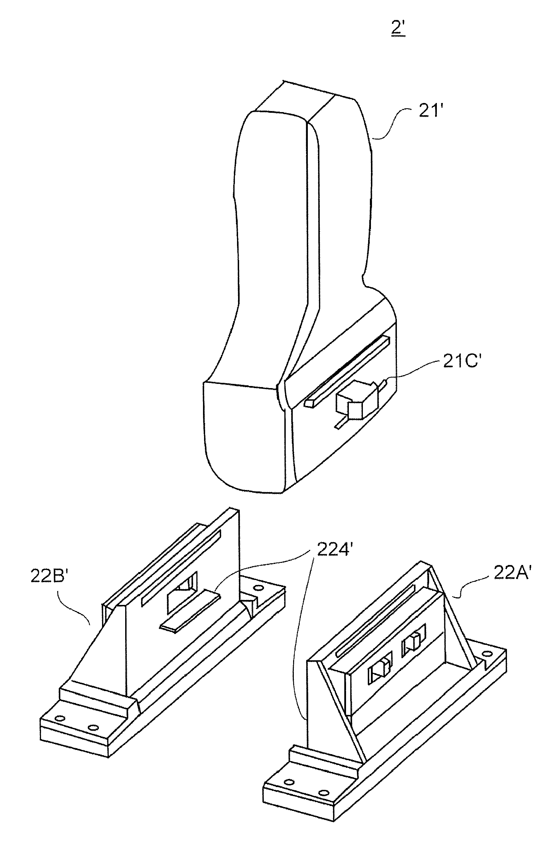

As a modified example of this structure, as shown in Fig. 29, the irradiation unit 22A' (or 22B') may be provided with a connector 224' (in Fig. 29, a male connector), and on the other hand, the ultrasonic wave probe 21' may be provided with a second connector (unillustrated; a female connector) so that, when the irradiation unit 22A' is fitted to the ultrasonic wave probe 21', the first connector 224' couples with the second connector through a hole 21c' in the ultrasonic wave probe 21'.

In that case, the electric power supplied from the main body (unillustrated) of the optoacoustic imaging device via the power cable (unillustrated) to the optoacoustic probe 2' is supplied via the second connector (unillustrated) and the first connector 224' to the LED light source.

In a case where the optoacoustic probe 2' is of a wireless type, since the LED light source consumes low electric power and can be battery-operated, the second connector may instead be connected to a battery incorporated in the ultrasonic wave probe.

### Tenth Embodiment

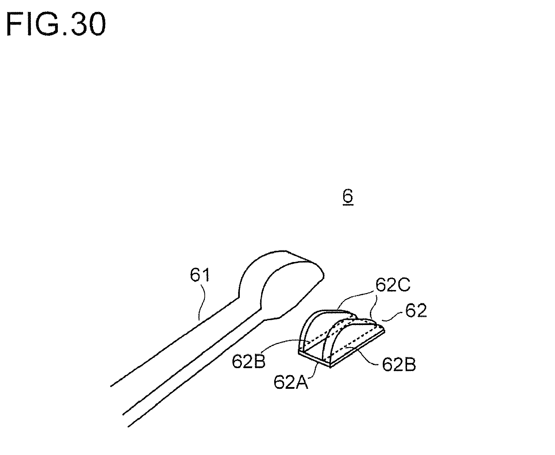

An outline of the structure of an optoacoustic probe according to a tenth embodiment of the present invention is shown in Fig. 30. The optoacoustic probe 6 shown in Fig. 30 includes an ultrasonic wave probe 61, which is a convex-type probe provided with an endoscope, and an irradiation unit 62 fitted on it. The ultrasonic wave probe 61 is used, for example, as a transesophageal probe.

The irradiation unit 62 includes, at opposite sides of a base 62A respectively, LED light sources 62B and light guide members 62C for guiding the light emitted from the LED light sources 62B.

The irradiation unit 62 is removably fitted on the ultrasonic wave probe 61 such that a tip end part of the ultrasonic wave probe 61 is held between the light guide members 62C from opposite sides.

### Other Modifications

Other conceivable modified examples are as follows. In the first and other embodiments, the front and rear faces of the ultrasonic wave probe 11 are each fitted with an irradiation unit; in addition, any side face may be removably fitted with another irradiation unit.

Another irradiation unit may additionally be removably fitted to any irradiation unit that is fitted to the ultrasonic wave probe. For example, in the first embodiment (Fig. 1), the upper light source cover 121 may be provided with an elevation 11A and a depression 11B like those provided in the ultrasonic wave probe 11 so that another irradiation unit can be fitted to the upper light source cover 121 by being slid along.

It should be understood that the embodiments by way of which the present invention is described herein allow for various modifications without departing from the spirit of the present invention.

For example, although in the embodiments described above the light source comprises an LED light source, it may instead comprise a semiconductor laser element, an organic light-emitting diode element, or the like.

## Claims

1. A probe for an optoacoustic imaging device, comprising:
an irradiator including a light-emitting semiconductor element light source that irradiates a tested object with light; and
a detector which detects an optoacoustic wave generated in the tested object as a result,
wherein the irradiator is removably fitted to the detector.

2. The probe according to claim 1, further comprising:
a slide mechanism which enables sliding of the irradiator relative to the detector; and
a lock which locks at a slide position up to which the sliding enabled by the slide mechanism is limited.

3. The probe according to claim 1, further comprising:
a fastening mechanism which fastens the irradiator to the detector, the fastening mechanism including two first hooks so biased as to come close together by an elastic member and a second hook, wherein inserting the second hook between the first hooks causes the first hooks to come apart and lock the second hook.

4. The probe according to claim 3,
wherein the fastening mechanism further includes an elevation and a depression which engage with each other when the second hook is locked by the first hooks.

5. The probe according to claim 1,
wherein the irradiator includes
releasable clamps which in a closed state hold the detector from opposite sides and
a lock which locks the releasable clamps in the closed state.

6. The probe according to claim 1, further comprising:
an attachment which is removably fitted to the detector,
wherein the irradiator is removably fitted to the attachment.

7. The probe according to claim 6, further comprising:
a slide mechanism which enables sliding of the irradiator relative to the attachment; and
a lock which locks at a slide position up to which the sliding enabled by the slide mechanism is limited.

8. The probe according to claim 6, further comprising:
a fastening mechanism which fastens the irradiator to the detector, the fastening mechanism including two first hooks so biased as to come close together by an elastic member and a second hook, wherein inserting the second hook between the first hooks causes the first hooks to come apart and lock the second hook.

9. The probe according to claim 8,
wherein the fastening mechanism further includes an elevation and a depression which engage with each other when the second hook is locked by the first hooks.

10. The probe according to claim 1,
wherein the irradiator includes
a first cover having an inner surface that, when the irradiator is fitted on a tip end part of the detector, makes close contact with the tip end portion, and
a second cover having a lock that, when the irradiator is fitted on the tip end part of the detector, locks on the tip end portion, and
the light-emitting semiconductor element light source is arranged around a circumference of the first cover while being enclosed by the first and second covers.

11. The probe according to any one of claims 1 to 10,
wherein a cable for supplying electric power from a main body of the optoacoustic imaging device to the light-emitting semiconductor element light source is connected to the irradiator.

12. The probe according to any one of claims 1 to 10, wherein
the detector has a first connector,
the irradiator has a second connector which, when the irradiator is fitted to the detector, couples with the first connector, and
electric power is supplied via the first and second connector to the light-emitting semiconductor element light source.

13. The probe according to any one of claims 1 to 12,
wherein the irradiator includes
a memory which stores identification information based on which the light-emitting semiconductor element light source can be identified,
a fitting detector which detects that the irradiator is fitted to the detector, and a transmitter which transmits the identification information based on a result of detection by the fitting detector.

14. The probe according to any one of claims 1 to 13,
wherein the light-emitting semiconductor element light source comprises a light-emitting diode element.

15. The probe according to any one of claims 1 to 13,
wherein the light-emitting semiconductor element light source comprises a light-emitting laser element.

16. The probe according to any one of claims 1 to 13,
wherein the light-emitting semiconductor element light source comprises an organic light-emitting diode element.

17. An optoacoustic imaging device comprising:
the probe according to any one of claims 1 to 16; and
an image generator which generates an optoacoustic image based on a detection signal from the detector.
